(19) Europäisches Patentamt / European Patent Office / Office européen des brevets

(11) **EP 2 366 387 B1**

(12) **EUROPEAN PATENT SPECIFICATION**

(45) Date of publication and mention of the grant of the patent:
**30.05.2018 Bulletin 2018/22**

(51) Int Cl.:
*A61K 9/51* *(2006.01)*    *A61K 49/18* *(2006.01)*
*C03B 37/01* *(2006.01)*    *C03C 13/00* *(2006.01)*
*A61K 9/20* *(2006.01)*

(21) Application number: **10194599.6**

(22) Date of filing: **10.12.2010**

(54) **Ceramic matrix for incorporating controlled release drugs, a tablet, method for obtaining the ceramic matrix and method for producing a tablet**

Keramikmatrix zur Aufnahme gesteuert freigesetzter Arzneien, Tablette, Verfahren zum Erhalt der Keramikmatrix und Verfahren zur Tablettenherstellung

Matrice céramique pour incorporer la libération contrôlée de médicaments, comprimé, procédé d'obtention de la matrice céramique et procédé de production d'un comprimé

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2009 BR 09068201**

(43) Date of publication of application:
**21.09.2011 Bulletin 2011/38**

(73) Proprietor: **Instituto Presbiteriano Mackenzie 01302-907 São Paulo - SP (BR)**

(72) Inventors:
• **Munhoz Junior, Antonio Hortencio 09050-410, Santo André (BR)**
• **Novickis, Richard Wagner 04520-013, São Paulo (BR)**
• **De Miranda, Leila Figueiredo 05014-002, São Paulo (BR)**
• **Faldini, Sonia Braunstein 01239-011, São Paulo (BR)**
• **Terence, Mauro Cesar 04116-060, São Paulo (BR)**
• **Ribeiro, Roberto Rodrigues São Paulo (BR)**

(74) Representative: **Heinemann, Christoph Geyer, Fehners & Partner mbB Patentanwälte Perhamerstrasse 31 80687 München (DE)**

(56) References cited:
**WO-A1-01/62232    WO-A1-2008/069561**

• **SUMIO SAKKA ET AL: "FIBERS FROM GELS", JOURNAL OF NON-CRYSTALLINE SOLIDS, NORTH-HOLLAND PHYSICS PUBLISHING. AMSTERDAM, NL, vol. 147/148, 1 October 1992 (1992-10-01), pages 394-403, XP000398428, ISSN: 0022-3093, DOI: DOI:10.1016/S0022-3093(05)80650-7**
• **THOMA K ALEX R: "Biodegradierbare gentamicin-depotimplantate aus beta-tricalciumphosphatkeramik", DIE PHARMAZIE, GOVI VERLAG PHARMAZEUTISCHER VERLAG GMBH, ESCHBORN, DE, vol. 46, no. 3, 1 January 1991 (1991-01-01), pages 198-202, XP002958006, ISSN: 0031-7144**

**Description**

Field of the Invention

The present invention refers to a method for preparing a ceramic nanosystem constructed for releasing medications in a controlled manner, in the form of a ceramic matrix, in the treatment of human beings and animals presenting an organic deficiency which requires the application of said medications.

Background of the Invention

Many of the drugs employed in the treatment of several diseases are indiscriminately distributed in several organs and tissues after administration thereof, which can cause inactivation of said drugs or undesirable effects not related to the pathological process. Besides, as a consequence of this wide distribution, for achieving the therapeutic concentration required in a certain organ or part of the organism, it is necessary to administer large amounts of the therapeutic agent.
Aiming at overcoming these drawbacks, there have been developed new drug-carrier systems to rationalize the medication therapy, leading to the reduction of the dose and of the undesired side effects, as well as stimulating the patient to adhere to the treatment.
The drug vectorization, based on Paul Ehrlich's the theory about the ability of tiny particles in carrying active molecules to the specific action sites, has been considered one of the major biopharmaceutical research lines of the last decades, taking part in a wide-range area, denominated nanotechnology, which quickly emerged in Brazil and in the world. It is a consensus that the use of nanostructured colloidal systems, such as the liposomes, nanoemulsions and polymeric nanoparticles, is an alternative which aims to alter the biodistribution of drugs after administration thereof by different routes.
The vector-oriented release system delivers, selectively, the drug to its action site, in order to offer the maximum therapeutic activity, prevent the degradation or inactivation during the transit until the target site, and protect the body from adverse reactions due to the inappropriate distribution (BANKER and RHODES, 1996).
Many pathologies present potential for treatment through drug vectorization such as, for example, parasite infections in cells of the endothelial reticulum system, diseases that affect the central nervous system, tumors, and the like. Specifically for cancer, the increase of the vascular permeability of the tumor tissue enables extravasation of the drug carriers presenting between 10 and 700 nanometers of diameter. This increase of the capillary permeability results from the poor formation of the neo-vasculature of the tumor tissues, which present gaps between the endothelial cells.
Thus, the application of the vectorized transport systems has potential to improve, for example, the chemotherapy of neoplasias. The effective use of said systems would, not only reduce the chemotherapeutic agent dose for a given degree of therapeutic answer, but also improve the opportunities for some cells which are typically resistant to certain drugs. Moreover, the chemotherapy application via these systems could reduce the complexity of the surgical manipulation, minimizing the severity of the cancer extension and/or reducing the residual volume.
Alternatively, the use of these systems, after the tumor has been reduced through surgery and/or radiation therapy, allows enhancing the probability of effectively eradicating the residual cancerous cells (GUPTA, 1990).
Another application of said technology might be noted in the international publication WO 2008/069561, which refers to a metal oxide hollow nanocapsule capable of carrying a drug adsorbed in its structure.
This prior art solution requires the provision of a nanocapsule surrounding the drug to be released in a predetermined organic medium, through the wall of the shell defined by said hollow nanocapsule. In this case, the drug is not incorporated in the metal oxide matrix itself, but enclosed in its interior.
The construction of the hollow nanocapsule requires specific and complex procedures, which demand sophisticated equipment and lead to high production costs.
Besides the above-cited drawback, the solution described in the international patent application mentioned above also requires that the metal oxide nanocapsule be surrounded by a silica coating to keep the drug contained in the interior of the nanocapsule, until the latter reaches the region of the organism able to remove the silica coating and allow the drug to be controllably and progressively released through the surrounding wall of the nanocapsule containing the drug.
The provision of the silica coating is fundamental to prevent undesired aggregations to the nanocapsule wall, which aggregations, without the provision of the coating, require the use of aqueous dispersions containing electrostatic stabilizers, surfactants, polymers, such as steric stabilizers and polymer modelers.
These aspects make it even more costly and complex the use of metal oxide nanocapsules encapsulating the drugs to be released.
WO 2008/069561 A1 discloses a method for preparing metal oxide hollow nanocapsules, which comprises a) dispersing metal oxyhydroxide particles in a water and alcohol mixture solution to prepare a metal oxyhydroxide dispersion solution, b) adding a silica precursor agent into the metal and alcohol mixture solution for sol-gel reaction to form the silica coating layer around the metal oxide particle, c) administering heat treatment to make the silica coating layered metal oxide hollow nanocapsules, and d) removing said silica coating layer.

WO 01/62232 A1 describes controlled release ceramic particles, wherein each of said particles has an active material substantially homogeneously dispersed throughout the particles and wherein the active material is capable of being released from said particles. It is further disclosed a process of preparing controlled release particles comprising preparing a reverse micelle solution by mixing a surfactant with an apolar solvent; preparing a precursor solution by dissolving a gel precursor, a catalyst, a condensing agent and a soluble active material in a polar solvent; preparing an emulsion by combining the reverse micelle solution and the precursor solution; forming and aging controlled release ceramic particles, wherein each of said particles has the active material substantially homogeneously dispersed throughout the particle and wherein the active material is capable of being released from said particle, by condensing the precursor in the emulsion.

Summary of the Invention

As a function of the drawbacks pointed above, the present invention has the object of obtaining a nanoceramic matrix, presenting a large specific area with controlled porosity and which is simple to produce at a relatively reduced cost. Through also simple and cost-effective methods, the present matrix can incorporate and retain, directly in its structure and without being surrounded by any external capsule, a large quantity of a pharmaceutical composition to be controllably released from the structure of the nanoceramic matrix to the human or animal organism to be treated.

According to a first aspect, said ceramic matrix comprises a structure formed by pseudoboehmite/y-alumina nanoparticles, presenting a specific area of 250-300 $m^2$/gram.

Further, said ceramic matrix of pseudoboehmite/y-alumina nanoparticles is used to produce a tablet, by mixing it, in an amount from 50% to 60% of the total tablet weight, with a pharmaceutical composition, generally comprising a drug defined by acyclovir or atenolol to complete the total tablet weight and which will be controllably released in a human or animal organism.

According to a further aspect of the invention, it is provided a method for obtaining said ceramic matrix, comprising the production of pseudoboehmite/y-alumina nanoparticles through the steps of:

- mixing an aluminium nitrate solution or aluminium chloride solution with a poly(vinyl alcohol) solution, forming a precursor solution;
- dripping the precursor solution in an ammonium hydroxide solution, forming a gel;
- ageing the gel, filtering and drying it at about 70°C for approximately 24 hours; and
- calcining the gel, at about 500°C to obtain a pseudoboehmite/y-alumina presenting a specific area of 250-300 $m^2$/gram.

The invention also refers to a method for producing a tablet, comprising, in a first phase, the production of a ceramic matrix of pseudoboehmite/y-alumina nanoparticles, through the steps of:

- mixing an aqueous aluminium nitrate solution or aqueous aluminium chloride solution (14%m) with a poly(vinyl alcohol) solution (8%m in water), forming a precursor solution;
- dripping the precursor solution in an ammonium hydroxide solution (28 %m), forming a gel;
- ageing the gel, filtering and drying it at about 70°C for approximately 24 hours; and
- calcining the gel, at about 500°C to obtain the ceramic matrix of pseudoboehmite/y-alumina presenting a specific area of 250-300 $m^2$/gram; and

in a second phase, mixing the ceramic matrix of pseudoboehmite/y-alumina, in an amount from 50% to 60% of the total tablet weight, with a pharmaceutical composition, in an amount to complement the total tablet weight, which will be controllably released in a human or animal organism.

Detailed Description of the Invention

As described above, the invention includes a method for preparing a ceramic matrix having a structure formed by pseudoboehmite/y-alumina nanoparticles, presenting a specific area of 250-300 $m^2$/gram.

This large specific area of the ceramic matrix of pseudoboehmite/y-alumina nanoparticles allows said matrix to incorporate, in small material volumes, usually defined in tablets to be ingested by the human being or animal, a large quantity of one or more drugs, generally provided with pharmaceutical compositions, incorporating a pharmaceutically acceptable filler and also at least one flow adjusting element and a lubricant agent which facilitates the final compression of the mixture defined by the ceramic matrix and pharmaceutical composition, for forming a tablet.

Thus, the invention allows obtaining a tablet comprising a ceramic matrix formed by pseudoboehmite/y-alumina nanoparticles, presenting a specific area of 250-300 $mg^2$/g and defining 50% to 60% of the total tablet weight; and a pharmaceutical composition, incorporated in said matrix, in a quantity completing the total tablet weight and to be controllably released in a human or animal organism, through the structural collapse of the tablet and the progressive release of the drug in relation to the pseudoboehmite/y-alumina nanoparticles.

In the preferred way, the pharmaceutical composition comprises a drug, a pharmaceutically acceptable filler, a flow adjusting element and a lubricant agent used in the compression phase of the formation of the tablet.

The drug used in the pharmaceutical composition is preferably defined by any of the acyclovir and atenolol compounds. In this particular case, the drug is provided in a dose of 100mg.

The pharmaceutically acceptable filler may be defined by starch, which is present in the pharmaceutical composition in an amount ranging from 20% to 30%.

The flow adjusting element is generally defined by silicon dioxide, which is present in an amount which ranges from 1.5% to 2% in relation to the total weight of the pharmaceutical composition.

The lubricant agent may be defined by magnesium stearate, which is present in the pharmaceutical composition in an amount ranging from 1.5% to 2%.

For obtaining a ceramic matrix to be used in the preparation of the tablets, it is applied a method which comprises the production of pseudoboehmite/y-alumina nanoparticles through the steps of:

- mixing an aluminium nitrate or aluminium chloride solution (14 %m) with a poly(vinyl alcohol) solution (8%m in water), forming a precursor solution;
- dripping the precursor solution in an ammonium hydroxide solution (18 %m), forming a gel;
- ageing the gel, filtering and drying it at about 70°C for approximately 24 hours; and
- calcining the gel, at about 500°C to obtain a ceramic matrix of pseudoboehmite/y-alumina presenting a specific area of 250-300 $m^2$/gram.

For the production of a tablet using the ceramic matrix obtained by the method described above, said ceramic matrix, in a quantity from 50% to 60% of the total tablet weight, is mixed with the above-defined pharmaceutical composition, to be controllably released in a human or animal organism.

Preparation of the tablets:

The procedures related to the production of tablets using the acyclovir drug will be commented below.

There were produced lots of acyclovir tablets adsorbed with the pseudoboehmite/y-alumina, for conducting in-process dissolution and control tests. Another lot was made using a physical mixture of the drug and of the ceramic material, in predefined proportions.

The preparation of the tablets was carried out by direct compression, through the rotating press (brand Lemaq - model Mini Express L.N.S.), according to a formulation as exemplified below:

```
Acyclovir = quantity sufficient to form a dose of 100 mg;

Starch - 30% of the total tablet formulation;


ablet formulation;
```

and

```
Magnesium stearate = 2% of the total tablet formulation;

Pseudoboehmite/γ-alumina = 50 to 60% of the total tablet
formulation.
```

Procedure:

Mixing the formulation components (ceramic matrix/pharmaceutical composition), except the magnesium stearate, in a V-shaped mixer (brand Lemaq - model M "V"), for at least 15 minutes.

Adding the magnesium stearate and mixing for at least 5 minutes. Transferring the mixture to the press-forming machine, with the aid of a scoop. Pressing the mixture in a 10mm punch. Proceeding to the in-process control tests (average mass, friability and hardness).

According to the invention, and considering the pseudoboehmite synthesis study previously carried out at the Material Characterization Laboratory of the Universidade Presbiteriana Mackenzie (Mackenzie Presbyterian University) (CARRIO, 2007; MUNHOZ JR, 2006), pseudoboehmites were synthesized from two precursors AlCl3 and Al(NO3)3.9H2O. The samples obtained were structurally analyzed and used as a support for the production of nanoparticulate systems containing bioactive molecules.

The evaluation of the systems produced as drug carriers was conducted through interaction tests, by using the techniques of UV-VIS spectrometry, scanning electron microscopy, X-ray diffraction and spectrophotometry in the infrared region.

Preparation of the pseudoboehmites

As already previously cited, the used reagents are aqueous aluminium nitrate solution (Al(NO3)3.9H2O), aqueous aluminium chloride solution, aqueous ammonium hydroxide solution (NH40H) (14%m and 28%m) and aqueous poly(vinyl alcohol) solution (8%m in water).

The poly(vinyl alcohol) solution was used to increase the viscosity of the aluminium nitrate or aluminium chloride solution.

The aluminium nitrate or aluminium chloride solution is mixed to the poly(vinyl alcohol) solution, forming a precursor solution which is then dripped in the ammonium hydroxide solution, forming a gel. After ageing the gel, this is filtered in a Buchner funnel and dried at 70°C for 24 hours.

For obtaining the $\gamma$-alumina, the pseudoboehmite should be calcined at 500°C. The X-ray diffraction is used to evaluate whether the $\gamma$-alumina was obtained. Depending on the result, the calcining temperature may be altered.

Incorporation of the drugs to the $\gamma$-alumina/ pseudoboehmite

The incorporation of the drugs to the ceramic matrix is conducted through the solubilization of the active principles in an appropriate solvent, followed by addition of the pseudoboehmite or $\gamma$-alumina. The mixture is maintained under constant agitation, at a determined temperature, during a given period of time.

All the experimental conditions are optimized with the purpose of searching for a greater interaction between the molecule and the ceramic material, in a shorter time and at a lower temperature for the test. After the incorporation of the active principles, the mixture is centrifuged and the supernatant analyzed by UV-VIS spectrophotometry, for determining the quantity of molecules which interacted with the ceramic material.

The dispersion is filtered and the resulting material is washed and dried to be used in posterior analytic procedures.

Interaction Test

Scanning electron microscopy, UV-VIS spectrophotometry, X-ray diffraction and infrared spectroscopy are the techniques used to confirm the interaction of the molecules with the two types of ceramic material.

Scanning electron microscopy: direct determination of the interaction process between drug/y-alumina or pseudoboehmite.

The scanning electron microscopy (SEM) is a technique which allows analyzing, visually, the spatial distribution of the particulate matters and, therefore, aids in analyzing the drug/pseudoboehmite interaction process of the drug/$\gamma$-alumina interaction, contributing to the analysis of the uniformity of its distribution and to the homogeneity of the inorganic crystals of the ceramic materials. The SEM provides information about the diameter of the particulate materials and about the reproducibility of the synthesis conditions, thus allowing adjusting and improving these procedures.

UV-VIS spectrometry: determination of the adsorption of the drug to the pseudoboehmite/y-alumina.

The quantification of the active component to be adsorbed by the ceramic material may be evaluated through the ultraviolet UV-VIS spectrophotometry, via calibration curve of each of the substances in the appropriate solvent for the adsorption test and in the more adequate wave length for each substance.

The optimization of the test conditions can be obtained by analyzing the conditions which most favor the adsorption. The parameters to be optimized are: total test time, temperature and the relation of concentration between the active principle and pseudoboehmite or $\gamma$-alumina.

Through the analysis by UV-VIS, it can be determined the amount of active component which was not adsorbed by the matrix and, by comparing these data with the previously obtained calibration curve, one can indirectly find the concentration of bioactive molecules which were adsorbed by the ceramic matrix.

Thus, it is possible to evaluate, for example, the pseudoboehmite/drug interaction and to know its adsorption yield.

X-ray diffraction: determination of the interaction process between drug and pseudoboehmite/γ-alumina

It should be emphasized that an X-ray diffraction equipment provides qualitative and quantitative information about the obtained structure and about the drug/pseudoboehmite or γ-alumina nanointeractions.

Absorption spectroscopy in the infrared region: determination of the interaction process between drug and pseudoboehmite/γ-alumina

The analysis by spectrophotometry in the infrared region can provide information about the adsorption mechanism, comparing the infrared spectra of the adsorbed drug and of the pure drug. It is possible to verify the absorption displacement of some groups of adsorbed drugs by influence of the ceramic material (WHITE & HEM, 1983).

**Claims**

1. A method for preparing pseudoboehmite, **characterized in that** it comprises the steps of:

   - mixing an aluminium nitrate or aluminium chloride solution with a poly(vinyl alcohol) solution, forming a precursor solution;
   - dripping the precursor solution into an ammonium hydroxide solution, forming a gel; and
   - ageing the gel, filtering and drying it by about 70°C for approximately 24 hours.

2. A method for obtaining a ceramic matrix, **characterized in that** it comprises the production of pseudoboehmite/γ-alumina nanoparticles through the steps of:

   - preparing pseudoboehmite according to the method of claim 1; and subsequently
   - calcining the gel, at about 500°C to obtain pseudoboehmite/γ-alumina presenting a specific area of 250-300 $m^2$/g.

3. A method for producing a tablet, **characterized in that** it comprises, in a first phase, the production of a ceramic matrix of pseudoboehmite nanoparticles, through the steps of:

   - mixing an aqueous aluminium nitrate solution or aqueous aluminium chloride solution (14 wt.-%) with a poly(vinyl alcohol) solution (8 wt.-% in water), forming a precursor solution;
   - dripping the precursor solution in an ammonium hydroxide solution (28 wt.-%), forming a gel;
   - ageing the gel, filtering and drying it at about 70°C for approximately 24 hours; and
   in a second phase, mixing the ceramic matrix of pseudoboehmite, in an amount from 50% to 60% of the total tablet weight, with a pharmaceutical composition, in an amount to complement the total tablet weight and to be controllably released in a human or animal organism; and
   - submitting the mixture ceramic matrix/pharmaceutical composition mixture to a conformation under a pressure sufficient to form the tablet.

4. A method for producing a tablet, **characterized in that** it comprises, in a first phase, the production of a ceramic matrix of pseudoboehmite/γ-alumina nanoparticles, through the steps of:

   - mixing an aqueous aluminium nitrate solution or aqueous aluminium chloride solution (14 wt.-%) with a poly(vinyl alcohol) solution (8 wt.-% in water), forming a precursor solution;
   - dripping the precursor solution in an ammonium hydroxide solution (28 wt.-%), forming a gel;
   - ageing the gel, filtering and drying it at about 70°C for approximately 24 hours;
   - calcining the gel, at about 500°C to obtain a ceramic matrix of pseudoboehmite/γ-alumina presenting a specific area of 250-300 $m^2$/gram; and
   in a second phase, mixing the ceramic matrix of pseudoboehmite/γ-alumina, in an amount from 50% to 60% of the total tablet weight, with a pharmaceutical composition, in an amount to complement the total tablet weight and to be controllably released in a human or animal organism; and

- submitting the mixture ceramic matrix/pharmaceutical composition mixture to a conformation under a pressure sufficient to form the tablet.

5. The method, as set forth in claim 3 or 4, **characterized in that** the step of mixing the ceramic matrix to the pharmaceutical composition comprises: mixing the ceramic matrix of pseudoboehmite/y-alumina with a drug, a pharmaceutically acceptable filler and a flow adjusting element, during at least 15 minutes; adding the lubricant agent; and mixing the ceramic matrix and the pharmaceutical composition for at least 5 minutes before submitting said mixture to the step of press-formation.

6. The method, as set forth in claim 3 or 4, **characterized in that** the pharmaceutical composition comprises a drug, a pharmaceutically acceptable filler, a flow adjusting element and a lubricant agent.

7. The method, as set forth in claim 5 or 6, **characterized in that** the drug is defined by any of the acyclovir and atenolol compounds, preferably **characterized in that** the drug is present in a therapeutic dose of 100 mg.

8. The method, as set forth in claim 5 or 6, **characterized in that** the pharmaceutically acceptable filler is defined by starch, which is present in an amount ranging from 20% to 30% by weight, in relation to the total tablet weight.

9. The method, as set forth in claim 5 or 6, **characterized in that** the flow adjusting element is defined by silicon dioxide, which is present in an amount ranging from 1.5% to 2% in relation to the total tablet weight and/or **characterized in that** the lubricant agent is defined by magnesium stearate, which is present in an amount ranging from 1.5% to 2% in relation to the total tablet weight.

## Patentansprüche

1. Verfahren zum Herstellen von Pseudoböhmit, **dadurch gekennzeichnet, dass** es die Schritte umfasst:

   - Mischen einer Aluminiumnitrat- oder Aluminiumchloridlösung mit einer Poly(vinylalkohol)lösung, Bilden einer Vorläuferlösung;
   - Tröpfeln der Vorläuferlösung in eine Ammoniumhydroxidlösung, Bilden eines Gels; und
   - Altern des Gels, Filtrieren und Trocknen für ungefähr 24 Stunden bei etwa 70°C.

2. Verfahren zum Erhalten einer keramischen Matrix, **dadurch gekennzeichnet, dass** es die Herstellung von Pseudoböhmit/$\gamma$-Aluminiumoxid-Nanopartikeln durch die Schritte umfasst:

   - Herstellen von Pseudoböhmit gemäß dem Verfahren nach Anspruch 1; und anschließend
   - Kalzinieren des Gels bei etwa 500°C, um Pseudoböhmit/$\gamma$-Aluminiumoxid zu erhalten, das eine spezifische Fläche von 250-300 $m^2$/g aufweist.

3. Verfahren zur Herstellung einer Tablette, **dadurch gekennzeichnet, dass** es in einer ersten Phase die Herstellung einer keramischen Matrix aus Pseudoböhmit-Nanopartikeln durch die Schritte umfasst:

   - Mischen einer wässrigen Aluminiumnitratlösung oder wässrigen Aluminiumchloridlösung (14 Gew.-%) mit einer Poly(vinylalkohol)lösung (8 Gew.-% in Wasser), Bilden einer Vorläuferlösung;
   - Tröpfeln der Vorläuferlösung in eine Ammoniumhydroxidlösung (28 Gew.-%), Bilden eines Gels;
   - Altern des Gels, Filtrieren und Trocknen bei etwa 70°C für ungefähr 24 Stunden; und
   in einer zweiten Phase Mischen der keramischen Matrix aus Pseudoböhmit in einer Menge von 50% bis 60% des Gesamttablettengewichts mit einer pharmazeutischen Zusammensetzung in einer Menge, die das Gesamttablettengewicht ergänzt und um sie kontrollierbar in einem menschlichen oder tierischen Organismus freizusetzen; und
   - Hinzufügen der Mischung aus keramischer Matrix/pharmazeutischer Zusammensetzung zu einer Konformation unter einem Druck, der ausreicht, um die Tablette zu bilden.

4. Verfahren zur Herstellung einer Tablette, **dadurch gekennzeichnet, dass** es in einer ersten Phase die Herstellung einer keramischen Matrix aus Pseudoböhmit/$\gamma$-Aluminiumoxid-Nanopartikeln durch die Schritte umfasst:

- Mischen einer wässrigen Aluminiumnitratlösung oder wässrigen Aluminiumchloridlösung (14 Gew.-%) mit einer Poly(vinylalkohol)lösung (8 Gew.-% in Wasser), Bilden einer Vorläuferlösung;

- Tröpfeln der Vorläuferlösung in eine Ammoniumhydroxidlösung (28 Gew.-%), Bilden eines Gels;

- Altern des Gels, Filtrieren und Trocknen bei etwa 70°C für ungefähr 24 Stunden;

- Kalzinieren des Gels bei etwa 500°C, um eine keramische Matrix aus Pseudoböhmit/$\gamma$-Aluminiumoxid zu erhalten, das eine spezifische Fläche von 250-300 m$^2$/g aufweist; und

in einer zweiten Phase Mischen der keramischen Matrix aus Pseudoböhmit/$\gamma$-Aluminiumoxid in einer Menge von 50% bis 60% des Gesamttablettengewichts mit einer pharmazeutischen Zusammensetzung in einer Menge, die das Gesamttablettengewicht ergänzt und um sie kontrollierbar in einem menschlichen oder tierischen Organismus freizusetzen; und

- Hinzufügen der Mischung aus keramischer Matrix/pharmazeutischer Zusammensetzung zu einer Konformation unter einem Druck, der ausreicht, um die Tablette zu bilden.

5. Verfahren nach Anspruch 3 oder 4, **dadurch gekennzeichnet, dass** der Schritt des Mischens der keramischen Matrix mit der pharmazeutischen Zusammensetzung umfasst: Mischen der keramischen Matrix aus Pseudoböhmit/$\gamma$-Aluminiumoxid mit einem Arzneimittel, einem pharmazeutisch annehmbaren Füllstoff und einem Flussanpassungselement für mindestens 15 Minuten; Zugabe des Schmiermittels; und Mischen der keramischen Matrix und der pharmazeutischen Zusammensetzung für mindestens 5 Minuten, bevor die Mischung dem Schritt der Pressformung unterzogen wird.

6. Verfahren nach Anspruch 3 oder 4, **dadurch gekennzeichnet, dass** die pharmazeutische Zusammensetzung ein Arzneimittel, einen pharmazeutisch annehmbaren Füllstoff, ein Flussanpassungselement und ein Gleitmittel umfasst.

7. Verfahren nach Anspruch 5 oder 6, **dadurch gekennzeichnet, dass** das Arzneimittel definiert ist durch Acyclovir- und Atenololverbindungen, vorzugsweise **dadurch gekennzeichnet, dass** das Arzneimittel in einer therapeutischen Dosis von 100 mg vorliegt.

8. Verfahren nach Anspruch 5 oder 6, **dadurch gekennzeichnet, dass** der pharmazeutisch annehmbare Füllstoff durch Stärke definiert ist, die in einer Menge im Bereich von 20 bis 30 Gew.-%, bezogen auf das Gesamttablettengewicht, vorhanden ist.

9. Verfahren nach Anspruch 5 oder 6, **dadurch gekennzeichnet, dass** das Flussanpassungselement durch Siliziumdioxid definiert ist, das in einer Menge im Bereich von 1,5% bis 2%, bezogen auf das Gesamttablettengewicht, vorliegt und/oder **dadurch gekennzeichnet ist, dass** das Schmiermittel durch Magnesiumstearat definiert ist, das in einer Menge im Bereich von 1,5% bis 2%, bezogen auf das Gesamttablettengewicht, vorhanden ist.

**Revendications**

1. Procédé de préparation de pseudoboehmite, **caractérisé en ce qu'**il comprend les étapes de :

- mélange d'une solution de nitrate d'aluminium ou de chlorure d'aluminium avec une solution d'alcool polyvinylique, de façon à former une solution de précurseur ;

- ajout goutte à goutte de la solution de précurseur dans une solution d'hydroxyde d'ammonium, de façon à former un gel ; et

- vieillissement du gel, filtration et séchage de celui-ci à environ 70 °C pendant approximativement 24 heures.

2. Procédé d'obtention d'une matrice de céramique, **caractérisé en ce qu'**il comprend la production de nanoparticules de pseudoboehmite/$\gamma$-alumine par l'intermédiaire des étapes de :

- préparation de pseudoboehmite selon le procédé de la revendication 1 ; et ensuite

- calcination du gel, à environ 500 °C pour obtenir de la pseudoboehmite/$\gamma$-alumine présentant une surface spécifique de 250 à 300 m$^2$/g.

3. Procédé de production d'un comprimé, **caractérisé en ce qu'**il comprend, dans une première phase, la production d'une matrice de céramique de nanoparticules de pseudoboehmite, par l'intermédiaire des étapes de :

- mélange d'une solution aqueuse de nitrate d'aluminium ou d'une solution aqueuse de chlorure d'aluminium (14 % en poids) avec une solution d'alcool polyvinylique (8 % en poids dans l'eau), de façon à former une solution de précurseur ;
- ajout goutte à goutte de la solution de précurseur dans une solution d'hydroxyde d'ammonium (28 % en poids), de façon à former un gel ;
- vieillissement du gel, filtration et séchage de celui-ci à environ 70 °C pendant approximativement 24 heures ; et dans une deuxième phase, mélange de la matrice de céramique de pseudoboehmite, dans une quantité de 50 % à 60 % du poids de comprimé total, avec une composition pharmaceutique, dans une quantité suffisante pour complémenter le poids total du comprimé et pour être libérée de façon contrôlable dans un organisme humain ou animal ; et
- soumission du mélange de matrice de céramique/ composition pharmaceutique à une conformation sous une pression suffisante pour former le comprimé.

4. Procédé de production d'un comprimé, **caractérisé en ce qu'**il comprend, dans une première phase, la production d'une matrice de céramique de nanoparticules de pseudoboehmite/γ-alumine, par l'intermédiaire des étapes de :

- mélange d'une solution aqueuse de nitrate d'aluminium ou d'une solution aqueuse de chlorure d'aluminium (14 % en poids) avec une solution d'alcool polyvinylique (8 % en poids dans l'eau), de façon à former une solution de précurseur ;
- ajout goutte à goutte de la solution de précurseur dans une solution d'hydroxyde d'ammonium (28 % en poids), de façon à former un gel ;
- vieillissement du gel, filtration et séchage de celui-ci à environ 70 °C pendant approximativement 24 heures ;
- calcination du gel, à environ 500 °C pour obtenir de la pseudoboehmite/γ-alumine présentant une surface spécifique de 250 à 300 $m^2$/g ; et

dans une deuxième phase, mélange de la matrice de céramique de pseudoboehmite/γ-alumine, dans une quantité de 50 % à 60 % du poids de comprimé total, avec une composition pharmaceutique, dans une quantité suffisante pour complémenter le poids total du comprimé et pour être libérée de façon contrôlable dans un organisme humain ou animal ; et
- soumission du mélange de matrice de céramique/ composition pharmaceutique à une conformation sous une pression suffisante pour former le comprimé.

5. Procédé selon la revendication 3 ou 4, **caractérisé en ce que** l'étape de mélange de la matrice de céramique avec la composition pharmaceutique comprend : le mélange de la matrice de céramique de pseudoboehmite/γ-alumine avec un médicament, une charge pharmaceutiquement acceptable et un élément d'ajustement d'écoulement, pendant au moins 15 minutes ; l'ajout de l'agent lubrifiant ; et le mélange de la matrice de céramique et de la composition pharmaceutique pendant au moins 5 minutes avant la soumission dudit mélange à l'étape de formation à la presse.

6. Procédé selon la revendication 3 ou 4, **caractérisé en ce que** la composition pharmaceutique comprend un médicament, une charge pharmaceutiquement acceptable, un élément d'ajustement d'écoulement et un agent lubrifiant.

7. Procédé selon la revendication 5 ou 6, **caractérisé en ce que** le médicament est défini comme étant l'un quelconque des composés acyclovir et aténolol, de préférence **caractérisé en ce que** le médicament est présent à une dose thérapeutique de 100 mg.

8. Procédé selon la revendication 5 ou 6, **caractérisé en ce que** la charge pharmaceutiquement acceptable est définie comme étant de l'amidon, qui est présent dans une quantité dans la plage de 20 % à 30 % en poids, par rapport au poids total de comprimé.

9. Procédé selon la revendication 5 ou 6, **caractérisé en ce que** l'élément d'ajustement d'écoulement est défini comme étant du dioxyde de silicium, qui est présent dans une quantité dans la plage de 1,5 % à 2 % par rapport au poids total de comprimé et/ou **caractérisé en ce que** l'agent lubrifiant est défini comme étant le stéarate de magnésium, qui est présent dans une quantité dans la plage de 1,5 % à 2 % par rapport au poids total de comprimé.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008069561 A
- WO 2008069561 A1

- WO 0162232 A1